# EUROPEAN PATENT APPLICATION

(11) **EP 1 939 174 A1**
(43) Date of publication of application: **02.07.2008**
(21) Application number: 06126928.8
(22) Date of filing: 21.12.2006
(51) Int. Cl.: C07D 205/08, A61K 31/397, A61K 47/48, A61P 3/06

(54) **Inclusion complex of ezetimibe and a cyclodextrin and processes in the preparation thereof**

(71) Applicant: LEK Pharmaceuticals d.d., 1526 Ljubljana (SI)
(72) Inventor: The designation of the inventor has not yet been filed
(74) Representative: Hodzar, Damjan

(57) **Abstract**

The invention belongs to the field of organic chemistry and relates to a novel inclusion complex of ezetimibe with a cyclodextrin. Provided is a process for the preparation of an inclusion complex of ezetimibe and a cyclodextrin and characterization of said prepared inclusion complex.

## Description

### Field of the Invention

The present invention belongs to the field of organic chemistry and relates to a novel inclusion complex of ezetimibe with a cyclodextrin, and methods of preparing such complexes.

### Background of the Invention

One of the most important physical properties of a pharmaceutical compound is its solubility in aqueous solution.

It is often preferable to deliver a pharmaceutical dose to a patient in aqueous solution and the solubility of the dose in the aqueous carrier is crucial for the efficacy of the composition.

In addition, solid oral pharmaceutical dosage forms need to be disintegrated in the digestive tract and the active ingredient should be completely dissolved in order to reach optimal physiological effect. Active ingredients that have a low solubility in water are generally not easily solubilised in the digestive tract, leading to problems with the delivery of the drug.

Therefore, there is a clear need to ensure that the active ingredient is soluble in aqueous solutions for all drug delivery methods. An otherwise potent drug can fail due to an inability to effectively deliver the active ingredient to the patent.

Certain potent active ingredients are either insoluble, or only slightly soluble in aqueous solutions. One such class of compounds is the cholesterol absorption inhibitors formed from a hydroxy substituted azetidinone. An example of a compound in this class is ezetimibe, which has the chemical name 1-4(fluorophenyl)-3-(*R*)-[3-(4-fluorophenyl)-3-(*S*)-hydroxypropyl]-4-(*S*)-(4-hydroxyphenyl)-2-azetidinone, and has following chemical structure:

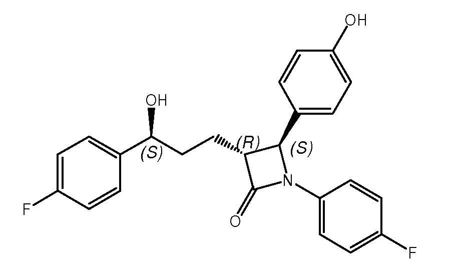

Ezetimibe is a potent cholesterol absorption inhibitor and appears as a white crystalline solid, freely soluble in alcohols and acetone, and practically insoluble in water, with a melting point of 164-166°C.

Studies have been carried out in the past to attempt to prepare crystalline forms of ezetimibe.

J. Org. Chem. 1999, 64, 3714 discloses the purification of ezetimibe by recrystallization in methyltertbutyl ether/heptane and methanol/water.

US patent 5886171 discloses the preparation of ezetimibe by crystallization from an aqueous solution of 2-propanol.

US patent 6207822 discloses the preparation of ezetimibe by crystallization from a mixture of methyltertbutyl ether and heptane, followed by a second crystallization from a mixture of methanol and water.

WO patent 2005/009955 discloses two crystalline forms and the processes for preparation thereof.

WO patent 2005/062897 discloses two crystalline forms and the processes for preparation thereof.

WO patent 2006/060808 discloses two crystalline forms and the processes for preparation thereof. It discloses also the micronized forms and ezetimibe having plate morphology.

None have successfully produced a reliable method of improving the solubility of ezetimibe in water.

It is known that many compounds can occur in different physical forms, including different crystal forms. This property is known as polymorphism and is a property associated with many molecules. Thus, a single molecule can give rise to a variety of crystalline forms having different and perhaps advantageous physical properties like solubility, melting point, infrared absorption spectrum, x-ray diffraction pattern and solid state NMR spectrum (Byrn, S.R.; Pfeiffer, R.R.; Stowell, J.G.; Solid-State Chemistry of Drugs, SSCI Inc. 1999).

However, it is not possible to predict in advance whether a different polymorph will give the desired properties.

As well as experimenting with different polymorphs of an active ingredient, other potential approaches to increase the solubility of an active ingredient include experimenting with the solvent systems, experimenting with the reaction conditions, considering pro-drugs or analogues, or forming complexes with other molecules.

Therefore, when faced with the problem of trying to increase the aqueous solubility of an active ingredient, there are many possible strategies that can be tried. Again, it is not possible to predict in advance whether a given strategy will be successful.

One approach is to consider forming a molecular complex between the active ingredient and another molecule (Byrn, S.R.; Pfeiffer, R.R.; Stowell, J.G.; Solid-State Chemistry of Drugs, SSCI Inc. 1999). Provided a suitable complexing molecule is chosen and suitable reaction conditions are discovered, an increase in solubility may be achieved but it is not possible to predict in advance whether a given complexing molecule will work, let alone determine the appropriate reaction conditions.

Therefore, there is a need for compounds, conditions, and/or processes that can enhance the solubility of active ingredients in general.

Specifically, there is a need to improve the solubility of ezetimibe. Any process or resultant pharmaceutical form that can achieve this would lead to a significant advantage by increasing bioavailability of drug delivery systems. Therefore, the need for soluble forms of ezetimibe is clearly needed.

The present invention successfully addresses the problems outlined above by preparing a soluble complex of ezetimibe and provides processes for preparing such complexes.

The present invention has found that a complex formed between ezetimibe and a cyclodextrin increases the solubility of ezetimibe.

### Summary of the Invention

According to a first aspect of the present invention, we provide a complex of ezetimibe or a pharmaceutically acceptable ester or analogue thereof, and a cyclodextrin.

For the first time, a complex of ezetimibe or a pharmaceutically acceptable ester or analogue thereof, and a cyclodextrin have been prepared, thereby increasing the solubility of the ezetimibe or the pharmaceutically acceptable ester or analogue thereof.

The present inventors discovered that a complex formed with a cyclodextrin increased the solubility of the active agent.

Cyclodextrins are cyclic oligosaccharides composed of multiple glucose residues. They are classified according to the number of sugar residues within the ring structure, α-cyclodextrin has 6 glucose residues, β-cyclodextrin has 7 glucose residues and γ-cyclodextrin has 8 glucose residues. Cyclodextrins can be modified by derivatisation to produce derivatives.

Cyclodextrins are cyclic compounds built up by six to eight glucopyranose units, linked by 1,4-glycosidic bonds into a macrocycle.

The inner cavity of a cyclodextrin is hydrophobic whereas the outer surface is hydrophilic. The hydrophobic interior is capable of encapsulating poorly soluble drugs. The hydrophilic exterior assists in solubilisation, so cyclodextrins can be useful adjuncts in pharmaceutical formulation.

In a preferred embodiment of the invention, the cyclodextrin or derivative thereof, is preferably an α-cyclodextrin, a β-cyclodextrin, a γ-cyclodextrin, or a derivative thereof.

The terms "cyclodextrin", "a cyclodextrin" or "the cyclodextrin" as used herein include cyclodextrins such as α-cyclodextrin, β-cyclodextrin, γ-cyclodextrin and derivatives thereof. It also includes substituted cyclodextrins, for example hydroxypropyl-cyclodextrins such as hydroxypropyl-β-cyclodextrin. Other substitutions are envisaged and included by the present invention. The term "cyclodextrin" may be substituted with the term "optionally substituted cyclodextrin" where appropriate.

Preferably, the cyclodextrin, is a β-cyclodextrin, more preferably hydroxypropyl-β-cyclodextrin.

The complex is preferably an inclusion complex. An inclusion complex can be a solid solution in which molecules of one compound occupy places in the crystal lattice of another compound. An inclusion compound can also be formed from molecular encapsulation, e.g. one compound trapped within another. Additionally, both forms of complex may be present in the solid complex, wherein a first compound is trapped within a second compound but also, the first and/or second compound may be trapped within a lattice formed by the complexed compounds. The term complex, or inclusion complex as discussed herein encompasses any or all of these definitions.

When an inclusion complex is formed between ezetimibe or a pharmaceutically acceptable ester or analogue thereof and a cyclodextrin, the cyclodextrin molecule may encapsulate the ezetimibe molecule and/or the ezetimibe molecule may occupy places in the crystal lattice of the cyclodextrins/ezetimibe-cyclodextrin complex.

The present invention is suitable for the increased solubilisation of hydroxy substituted azetidinones, as described in US5846966, the contents of which are hereby incorporated by reference. The present invention is particularly applicable to ezetimibe, together with its isomers, analogues and esters thereof.

The term "ezetimibe or a pharmaceutically acceptable ester or analogue thereof" as used herein is intended to cover ezetimibe itself as well as isomers, analogues and esters of ezetimibe.

Compounds of the present invention have at least two asymmetric carbon atoms, and in the case of ezetimibe itself, three asymmetric carbon atoms. All isomers, including enantiomers and diastereomers are encompassed by the present invention. The invention includes complexes comprising both D and L enantiomers and diastereoisomers in both the pure form and in admixtures, including racemic mixtures. Isomers can be prepared using conventional techniques, either by reacting chiral starting materials or by separating isomers. Isomers may also include geometric isomers, e.g. where a double bond is present.

The hydroxy groups of the compounds of the invention can protected by an ester group to give a pro-drug which can be cleaved *in vivo* by a biological method such as hydrolysis. Examples of suitable protecting ester groups which can be cleaved *in vivo* include 1-(acyloxy)"lower alkyl groups", for example, 1-("lower aliphatic acyl"oxy)"lower alkyl groups" such as formyloxymethyl, acetoxymethyl, dimethylamino-acetoxymethyl, propionyloxymethyl, butyryloxymethyl, pivaloyloxymethyl, valeryloxymethyl, isovaleryloxymethyl, hexanoyloxymethyl, 1-formyloxyethyl, 1-acetoxyethyl, 1-propionyloxyethyl, 1-butyryloxyethyl, 1-pivaloyloxyethyl, 1-valeryloxyethyl, 1-isovaleryloxyethyl, 1-hexanoyloxyethyl, 1-formyloxypropyl, 1-acetoxypropyl, 1-propionyloxypropyl, 1-butyryloxypropyl, 1-pivaloyloxypropyl, 1-valeryloxypropyl, 1-isovaleryloxypropyl, 1-hexanoyloxypropyl, 1-acetoxybutyl, 1-propionyloxybutyl, 1-butyryloxybutyl, 1-pivaloyloxybutyl, 1-acetoxypentyl, 1-propionyloxypentyl, 1-butyryloxypentyl, 1-pivaloyloxypentyl and 1-pivaloyl-oxyhexyl groups, 1-("cycloalkyl"carbonyloxy)"lower alkyl groups" such as cyclopentylcarbonyloxymethyl, cyclohexylcarbonyloxymethyl, 1-cyclopentylcarbonyloxyethyl, 1-cyclohexylcarbonyloxyethyl, 1-cyclopentylcarbonyloxypropyl, 1-cyclohexyl-carbonyloxypropyl, 1-cyclopentylcarbonyloxybutyl and 1-cyclohexyl-carbonyloxybutyl groups, and 1-("aromatic acyl"oxy)"lower alkyl groups" such as benzoyloxymethyl groups; (lower alkoxycarbonyloxy)alkyl groups such as methoxycarbonyloxymethyl, ethoxycarbonyloxymethyl, propoxycarbonyloxymethyl, isopropoxycarbonyloxymethyl, butoxycarbonyloxymethyl, isobutoxycarbonyloxymethyl, pentyloxycarbonyloxymethyl, hexyloxycarbonyloxymethyl, cyclohexyloxycarbonyloxymethyl, cyclohexyloxycarbonyloxy(cyclohexyl)methyl, 1-(methoxycarbonyloxy)ethyl, 1-(ethoxycarbonyloxy)ethyl, 1-(propoxycarbonyloxy)-ethyl, 1-(isopropoxycarbonyloxy)ethyl, 1-(butoxycarbonyloxy)ethyl, 1-(isobutoxycarbonyloxy)ethyl, 1-(tert-butoxycarbonyloxy)ethyl, 1-(pentyloxycarbonyloxy)ethyl, 1-(hexyloxycarbonyloxy)ethyl, 1-(cyclopentyloxycarbonyloxy)ethyl, 1-(cyclopentyloxycarbonyloxy)propyl, 1-(cyclohexyloxycarbonyloxy)propyl, 1-(cyclopentyloxycarbonyloxy)butyl, 1-(cyclohexyloxycarbonyloxy)butyl, 1-(cyclohexyloxycarbonyloxy)ethyl, 1-(ethoxycarbonyloxy)propyl, 2-(methoxycarbonyloxy)ethyl, 2-(ethoxycarbonyloxy)-ethyl, 2-(propoxycarbonyloxy)ethyl, 2-(isopropoxycarbonyloxy)ethyl, 2-(butoxycarbonyloxy)ethyl, 2-(isobutoxycarbonyloxy)ethyl, 2-(pentyloxycarbonyloxy)ethyl, 2-(hexyloxycarbonyloxy)ethyl, 1-methoxycarbonyloxy)propyl, 1-(ethoxycarbonyloxy)propyl, 1-(propoxycarbonyloxy)propyl, 1-(isopropoxycarbonyloxy)propyl, 1-(butoxycarbonyloxy)propyl, 1-(isobutoxycarbonyloxy)propyl, 1-(pentyloxycarbonyloxy)propyl, 1-(hexyloxycarbonyloxy)propyl, 1-(methoxycarbonyloxy)butyl, 1-(ethoxycarbonyloxy)butyl, 1-(propoxycarbonyloxy)butyl, 1-(isopropoxycarbonyloxy)butyl, 1-(butoxycarbonyloxy)butyl, 1-(isobutoxycarbonyloxy)butyl, 1-(methoxycarbonyloxy)pentyl, 1-(ethoxycarbonyloxy)pentyl, 1-(methoxycarbonyloxy)hexyl and 1-(ethoxycarbonyloxy)hexyl groups; and oxodioxolenyl-methyl groups such as (5-phenyl-2-oxo-1,3-dioxolen-4-yl)methyl, [5-(4-methylphenyl)-2-oxo-1,3-dioxolen-4-yl]methyl, [5-(4-methoxyphenyl)-2-oxo-1,3-dioxolen-4-yl]methyl, [5-(4-fluorophenyl)-2-oxo-1,3-dioxolen-4-yl]methyl, [5-(4-chlorophenyl)-2-oxo-1,3-dioxolen-4-yl]methyl, (2-oxo-1,3-dioxolen-4-yl)methyl, (5-methyl-2-oxo-1,3-dioxolen-4-yl)methyl, (5-ethyl-2-oxo-1,3-dioxolen-4-yl)methyl, (5-propyl-2-oxo-1,3-dioxolen-4-yl)methyl, (5-isopropyl-2-oxo-1,3-dioxolen-4-yl)methyl and (5-butyl-2-oxo-1,3-dioxolen-4-yl)methyl groups: "phthalidyl groups" such as phthalidyl, dimethylphthalidyl and dimethoxyphthalidyl groups: the above-described "lower aliphatic acyl groups": the above-described "aromatic acyl groups": "half ester salt residue of succinic acid": "phosphate salt residues": "ester forming residues such as with amino acids": carbamoyl groups: carbamoyl groups substituted with 1 or 2 lower alkyl groups: and "1-(acyloxy)alkyloxycarbonyl groups" such as pivaloyloxymethyloxycarbonyl, of which the "carbonyloxyalkyl groups" are preferred.

The present inventors have discovered that an inclusion complex between ezetimibe or a pharmaceutically acceptable ester or analogue thereof, and a cyclodextrin solubilises the ezetimibe. This is surprising because the ezetimibe or the pharmaceutically acceptable ester or analogue thereof, is insoluble in water, and cyclodextrins are soluble in water. One would therefore expect that both ezetimibe or a pharmaceutically acceptable ester or analogue thereof, and the cyclodextrin cannot be present in the same solution, which is the necessary condition for the formation of an inclusion complex.

From many prior experiments, it has been found that the addition of a water solution of a cyclodextrin precipitates ezetimibe out of the solution, making formation of an inclusion complex impossible.

Indeed, when trying to form an inclusion complex with ezetimibe or a pharmaceutically acceptable ester or analogue thereof, many different solvents were tried by the present inventors. In most cases, one or other of the compounds precipitated out of solution during a stage of the process, for example mixing the solutions or lyophilization. Examples of solvents that proved to be unsuitable include methanol, ethanol, 1-propanol and 2-propanol, which were all tried with a cyclodextrin solution (HPBCD - hydroxypropyl β-cyclodextrin) in water.

Therefore, it could not have been predicted that it would be possible to form an inclusion complex between ezetimibe or a pharmaceutically acceptable ester or analogue thereof, and a cyclodextrin.

According to a preferred embodiment, the present invention provides an inclusion complex of ezetimibe or a pharmaceutically acceptable ester or analogue thereof, and a cyclodextrin.

Preferably, the present invention provides an inclusion complex of ezetimibe or a pharmaceutically acceptable ester or analogue thereof and β-cyclodextrin. Even more preferably, the present invention provides an inclusion complex of ezetimibe or a pharmaceutically acceptable ester or analogue thereof and hydroxypropyl-β-cyclodextrin. Particularly preferably, the present invention provides an inclusion complex of ezetimibe and hydroxypropyl-β-cyclodextrin.

The present invention also discloses selected solvents which can be used to separately dissolve ezetimibe or a pharmaceutically acceptable ester or analogue thereof, and a cyclodextrin, followed by mixing both solutions together to form the inclusion complex of the present invention without precipitating out either compound. Such a mixture can then be transformed by further treatment to obtain a solid complex.

Therefore, according to a further aspect of the present invention, we provide a method for the preparation of an inclusion complex of ezetimibe or a pharmaceutically acceptable ester or analogue thereof and a cyclodextrin comprising:
a) preparation of a solution of ezetimibe or a pharmaceutically acceptable ester or analogue thereof in a water miscible organic solvent;
b) preparation of a solution of a cyclodextrin in water;
c) combining the prepared solution of the ezetimibe or a pharmaceutically acceptable ester or analogue thereof, in the water miscible organic solvent, with the solution of the cyclodextrin in water.

For the first time, a method is provided which can successfully form an inclusion complex between ezetimibe or a pharmaceutically acceptable ester or analogue thereof, and a cyclodextrin. The method overcomes the considerable problems associated with the differing solubilities of ezetimibe or its pharmaceutically acceptable esters or analogues thereof, and cyclodextrins.

This discovery could not have been predicted because of this differing solubility and allows formation of the novel inclusion complex of the invention, which successfully increases the solubility of ezetimibe or its pharmaceutically acceptable esters or analogue thereofs, in water.

The first step involves preparation of a solution of ezetimibe or a pharmaceutically acceptable ester or analogue thereof, in a water miscible organic solvent. The organic solvent is selected from suitable solvents that will mix with the cyclodextrin solvent. Suitable solvents include any water miscible solvent that dissolves ezetimibe or the pharmaceutically acceptable esters or analogues thereof. Preferably, the solvents should also be able to be freeze dried.

According to a preferred aspect of the present invention, suitable organic solvents include alcohols or acetone. According to a particularly preferred embodiment, tert-butanol is used as the organic solvent.

Preferably, the ezetimibe or the pharmaceutically acceptable ester or analogue thereof, should be dissolved in the concentration range of 24 to 30 mg/ml, more preferably 25 mg/ml.

The temperature of the water miscible organic solvent should preferably be in range of between 0°C and 83°C, more preferably between 15°and 35°C, and most preferably between 25°and 30°C.

In the second step of the method, a solution of the cyclodextrin in water is prepared. The method may suitably use water. Other solvents that can both dissolve a cyclodextrin and are miscible with the organic solvent that dissolves the ezetimibe or the pharmaceutically acceptable esters or analogues thereof, can also be used, but water is preferred.

The temperature of the water or other appropriate solvent should preferably be in range of between 0°C and 83°C, more preferably between 15°and 35°C, and most preferably between 25°and 30°C.

Preferably, the cyclodextrin compound should be dissolved in the concentration range of :_300 to 400 mg/ml, more preferably 330 to 340 mg/ml.

The ezetimibe or the pharmaceutically acceptable ester or analogue thereof solution, and the cyclodextrin solution may be mixed in specific ratios. Suitable ratios include a molar ratio of 10:1, 8:1, 5:1, 3:1, 2:1, 1:1, 1:2, 1:3, 1:5, 1:8 or 1:10.

A more preferred molar ratio between ezetimibe or the pharmaceutically acceptable ester or analogue thereof, and the cyclodextrin in the two solutions is 1:1 - 1:2, more preferably 1:1.1 - 1:1.8, even more preferably 1:1.2 - 1:1.6, and yet more preferably 1:1.2 - 1:1.4. A particularly preferred ratio of ezetimibe or the pharmaceutically acceptable ester or analogue thereof, to the cyclodextrin is 1:1.3.

According to a preferred embodiment, the two solutions are stirred once combined to help avoid either compound coming out of solution. The stirring helps to prevent accidental precipitation of the ezetimibe or the pharmaceutically acceptable ester or analogue thereof. It is particularly preferred when the stirring is vigorous. Therefore, a preferable step d) comprises (vigorously) stirring the combined solution.

Preferably, the combined solution is lyophilised once the inclusion complex is formed. This involves freezing and freeze-drying. This step usually lasts for 3-5 hours until the solvent evaporates. Therefore, a preferable step e) comprises freezing and freeze-drying the solution.

The product is an inclusion complex between ezetimibe or the pharmaceutically acceptable ester or analogue thereof, and the cyclodextrin.

The present invention is also directed to pharmaceutical compositions or preparations comprising an inclusion complex as described herein or a complex formed from the methods described herein together with a pharmaceutically acceptable carrier.

These preparations are prepared by a well-known method using additives such as excipients (which may include organic excipients such as sugar derivatives, e.g., lactose, sucrose, glucose, mannitol and sorbitol; starch derivatives, e.g., corn starch, potato starch, α-starch and dextrin; cellulose derivatives, e.g., crystalline cellulose; gum arabic; dextran; and pullulan; and inorganic excipients such as silicate derivatives, e.g., light silicic anhydride, synthetic aluminum silicate, calcium silicate and magnesium aluminate meta-silicate; phosphates, e.g., calcium hydrogenphosphate; carbonates, e.g., calcium carbonate; and sulfates, e.g., calcium sulfate), lubricants (for example, stearic acid, stearic acid metal salts such as calcium stearate and magnesium stearate; talc; colloidal silica; waxes such as bee gum and spermaceti; boric acid; adipic acid; sulfates such as sodium sulfate; glycol; fumaric acid; sodium benzoate; DL-leucine; fatty acid sodium salts; lauryl sulfates such as sodium lauryl sulfate and magnesium lauryl sulfate; silicic acids such as silicic anhydride and silicic acid hydrate; and the above starch derivatives), binders (for example, hydroxypropyl cellulose, hydroxypropyl methyl cellulose, polyvinylpyrrolidone, Macrogol and compounds similar to the above excipients), disintegrants (for example, cellulose derivatives such as low-substituted hydroxypropyl cellulose, carboxymethyl cellulose, calcium carboxymethyl cellulose and internally bridged sodium carboxymethyl cellulose; and chemically modified starch/cellulose such as carboxymethyl starch, sodium carboxymethyl starch and bridged polyvinylpyrrolidone), stabilizers (which may include para-oxy benzoates such as methyl paraben and propyl paraben; alcohols such as chlorobutanol, benzyl alcohol and phenylethyl alcohol; benzalkonium chloride; phenols such as phenol and cresol; thimerosal; dehydroacetic acid; and sorbic acid), corrigents (which may include sweeteners, souring agents, flavors, etc. usually used), diluents, etc.

Examples of pharmaceutical compositions include any solid (tablets, pills, capsules, granules, etc.) or liquid (solutions, suspensions or emulsions) with a suitable composition or oral, topical or parenteral administration, and they may contain the pure complex or in combination with any carrier or pharmacologically active compounds. These compounds may need to be sterile when administered.

The present invention also encompasses the use of an inclusion complex as described herein or a complex formed from the methods described herein in the manufacture of a medicament.

Specifically, the present invention encompasses the use of an inclusion complex of ezetimibe or a pharmaceutically acceptable ester or analogue thereof, and a cyclodextrin, preferably, a β-cyclodextrin, even more preferably a hydroxypropyl-β-cyclodextrin, in the manufacture of a medicament.

Even more specifically, the present invention encompasses the use of an inclusion complex of ezetimibe and a cyclodextrin, preferably, a β-cyclodextrin, even more preferably a hydroxypropyl-β-cyclodextrin, in the manufacture of a medicament.

The present invention also extends to the use of a therapeutically effective amount of an inclusion complex of a cyclodextrin and ezetimibe or a pharmaceutically acceptable ester or analogue thereof as described herein, in the manufacture of a medicament.

The present invention also encompasses the use of an inclusion complex of the present invention as described herein or a complex formed from the methods described herein in the manufacture of a medicament for the prophylaxis or treatment of heart disease, coronary heart disease, atherosclerotic coronary heart disease or for the inhibition of cholesterol absorption.

The present invention also encompasses the use of a therapeutically effective amount of an inclusion complex of the present invention as described herein or a complex formed from the methods described herein in the manufacture of a medicament for the prophylaxis or treatment of heart disease, coronary heart disease, atherosclerotic coronary heart disease or for the inhibition of cholesterol absorption.

The present invention also extends to the complexes of the present invention for use in a method of treatment, and to the use of the complexes of the present invention in the preparation of a composition for the treatment of heart disease, coronary heart disease, or atherosclerotic coronary heart disease, or for the inhibition of cholesterol absorption.

The present invention also extends to a method for the prophylaxis or treatment of a patient suffering from heart disease, coronary heart disease, or atherosclerotic coronary heart disease, comprising administering a pharmaceutically effective amount of an inclusion complex of the present invention as described herein.

The present invention also extends to a method of inhibiting cholesterol absorption in a patient, comprising administering a pharmaceutically effective amount of an inclusion complex of the present invention as described herein.

The correct dosage of the inclusion complex will vary according to the particular formulation, the mode of application and the particular type of treatment being considered. Other factors like age, body weight, sex, diet, time of administration, rate of excretion, condition of the host, drug combination, reaction sensitivities, and severity of the disease shall be taken into account.

The inclusion complex and compositions of the present invention may be used with other drugs to provide a combination therapy. The other drugs may form part of the same composition, or be provided as a separate composition for administration at the same time or a different time.

A preferred combination therapy uses a combination of ezetimibe or a pharmaceutically acceptable ester or analogue thereof, and simvastatin.

### Brief Description of the Figures

Figure 1: X-Ray diffraction pattern of an inclusion complex of ezetimibe and hydroxypropyl-β-cyclodextrin, prepared according to Example 1
Figure 2: solid state ¹³C NMR spectrum of ezetimibe
Figure 3: solid state ¹³C NMR spectrum of hydroxypropyl-β-cyclodextrin
Figure 4: solid state ¹³C NMR spectrum of the mixture of ezetimibe and hydroxypropyl-β-cyclodextrin, prepared according to Example 1
Figure 5: solid state ¹³C NMR spectrum of inclusion complex between ezetimibe and hydroxypropyl-β-cyclodextrin, prepared according to Example 1
Figure 6: DSC thermogram of crystalline ezetimibe
Figure 7: DSC thermogram of hydroxypropyl-β-cyclodextrin
Figure 8: DSC thermogram of the inclusion complex between ezetimibe and hydroxypropyl-β-cyclodextrin, prepared according to Example 1
Figure 9: IR spectrum of crystalline ezetimibe
Figure 10: IR spectrum of the inclusion complex between ezetimibe and hydroxypropyl-β-cyclodextrin, prepared according to Example 1

### Detailed Description of Invention

The following example is offered to illustrate aspects of the present invention, and is not intended to limit or define the present invention in any manner.

### Example 1.

Separate solutions of 1.04 g ezetimibe in 42 ml of *tert*-butanol and 4.06g hydroxypropyl-β-cyclodextrin (source: Hengshan Kaida Fine Chemical CO) in 12 ml of water were prepared. These solutions were then combined and vigorously stirred until a clear solution was obtained. The resulting solution was freeze dried at -1 °C for 5 hours to give ~5 g of a fluffy material which was characterized as an ezetimibe hydroxypropyl-β-cyclodextrin complex, assay 18.07% ezetimibe (which represents a ratio of 5/8 mmol to 1 mmol HPBCD, MW 1163).

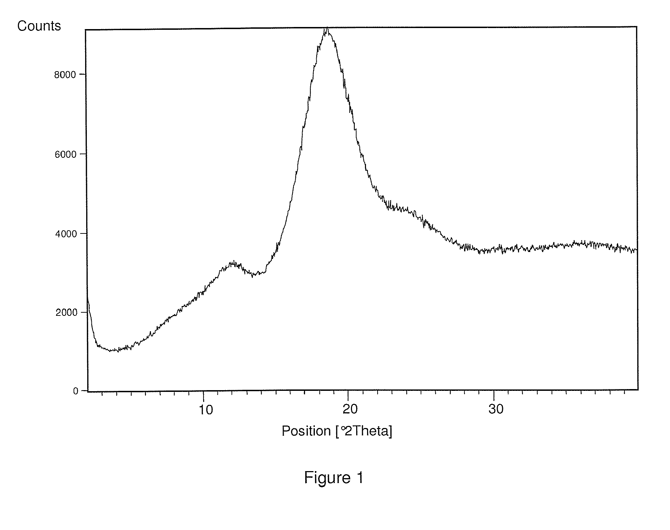

Figure 1 shows an X-Ray diffraction pattern of an inclusion complex of ezetimibe and hydroxypropyl-β-cyclodextrin, prepared according to Example 1.

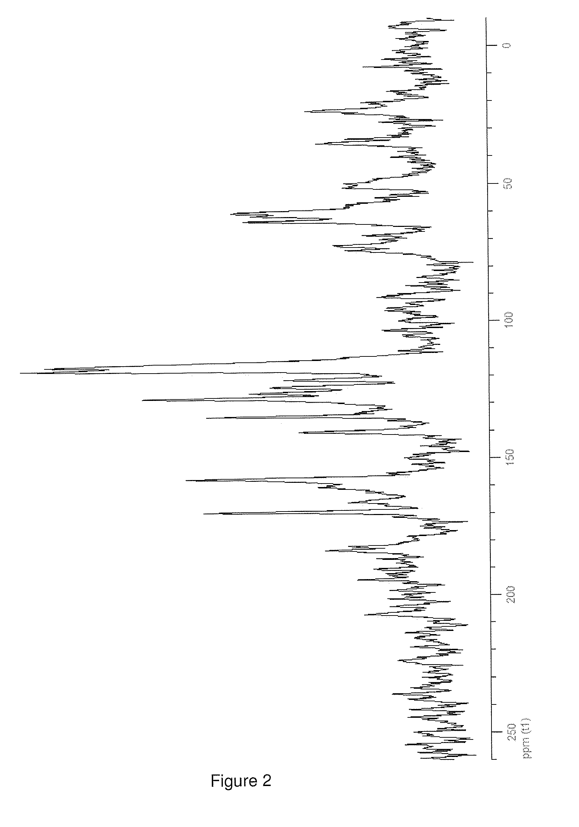

Figure 2 shows a solid state ¹³C NMR spectrum of ezetimibe alone.

The SS CPMAS 150MHz ¹³C spectrum were recorded on Varian Unity Inova 600 MHz NMR spectrometer with 3.2 mm NB Double Resonance HX MAS Solids Probe, spinning rate for magic angle spinning 10000Hz. Pulse sequence: Varian variable amplitude cross polarization (vacp), cross polarization contact time 5ms, no suppressing of spinning side bands. ¹³C NMR Chemical shifts have reference to external hexamethyl benzene.

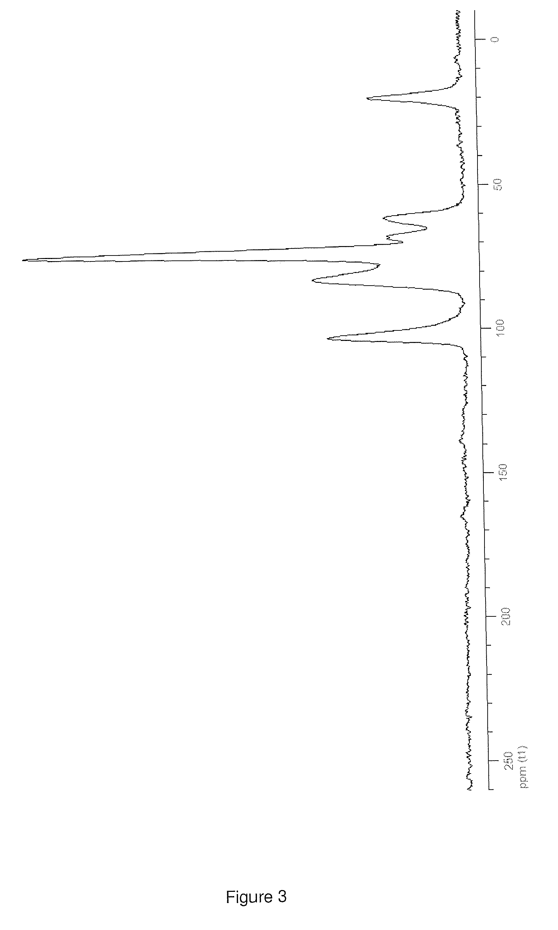

Figure 3 shows a solid state ¹³C NMR spectrum of hydroxypropyl-β-cyclodextrin alone.

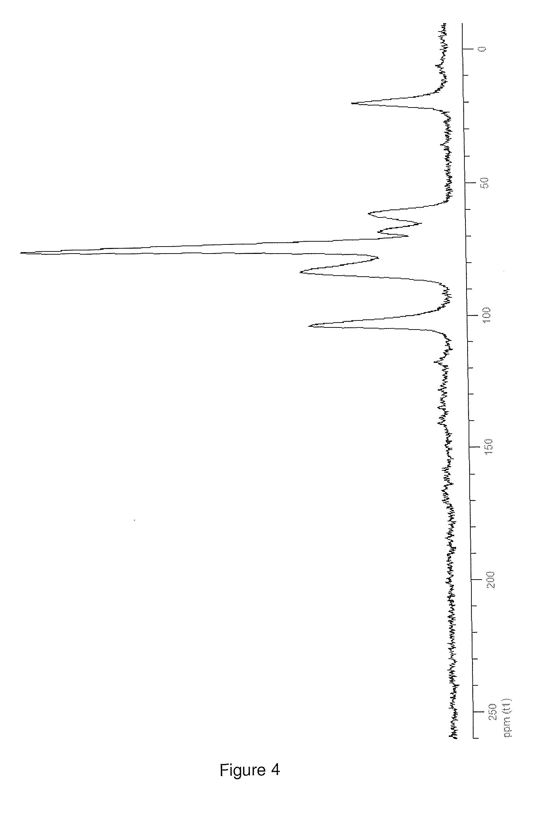

Figure 4 shows a solid state ¹³C NMR spectrum of the mixture of ezetimibe and hydroxypropyl-β-cyclodextrin. This is a physical mixture of ezetimibe and hydroxypropyl-β-cyclodextrin prepared by mixing the exact amounts of the solid components as used in Example 1. The compounds were mixed so as to form as homogenous a mixture as possible. This shows that an inclusion complex has not formed because the spectrum shows no significant peak changes from Figure 3.

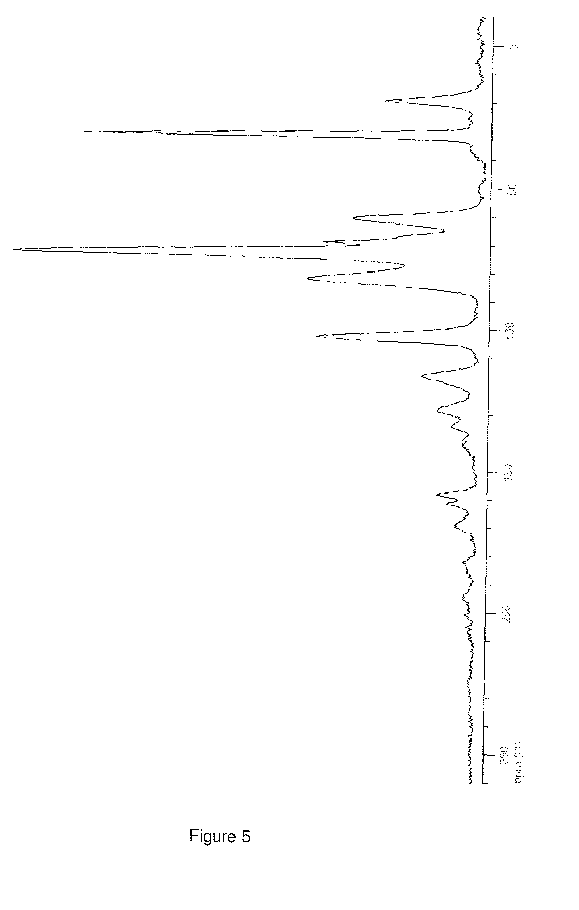

Figure 5 shows a solid state ¹³C NMR spectrum of the inclusion complex between ezetimibe and hydroxypropyl-β-cyclodextrin, prepared according to Example 1. It can be clearly seen that, in contrast to Figure 4, the complex has given rise to a unique spectrum clearly showing the complexing of the ezetimibe and hydroxypropyl-β-cyclodextrin.

The characterization of the obtained inclusion complex is clearly demonstrated with the solid state ¹³C NMR spectroscopy in Figures 2-5. The spectrum show the significant difference between the characteristic peaks found in the spectrum of a simple mixture of ezetimibe and hydroxypropyl-β-cyclodextrin (shown in Figure 4), and the characteristic peaks found for the inclusion complex, prepared according the procedure disclosed in the present invention (Figure 5).

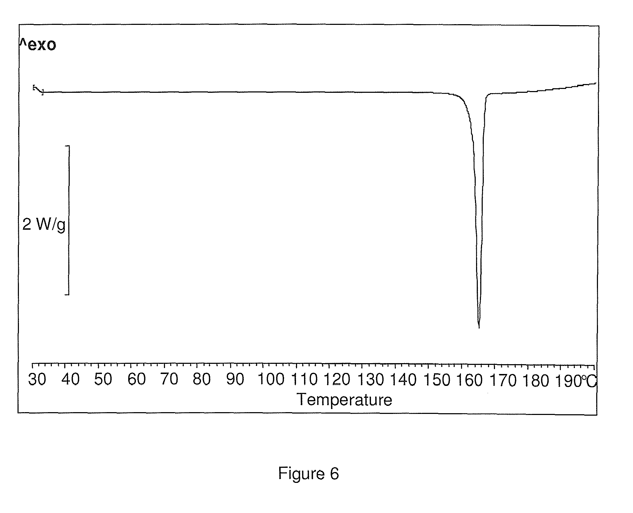

Figure 6 shows a DSC thermogram of crystalline ezetimibe. It shows an endothermic peak (melting point) at 165ºC which is representative for crystalline ezetimibe.

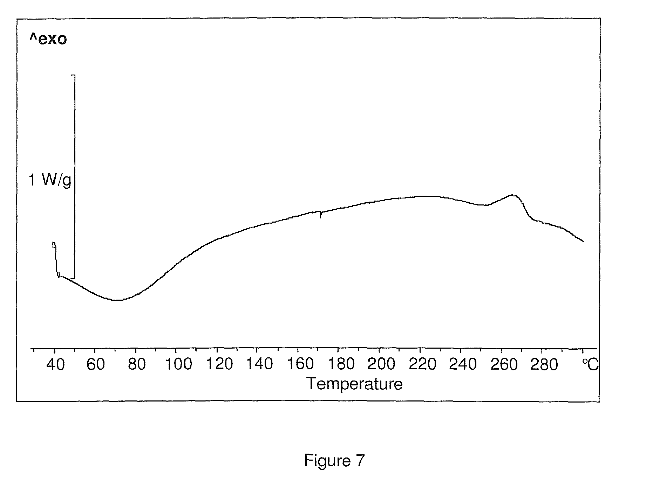

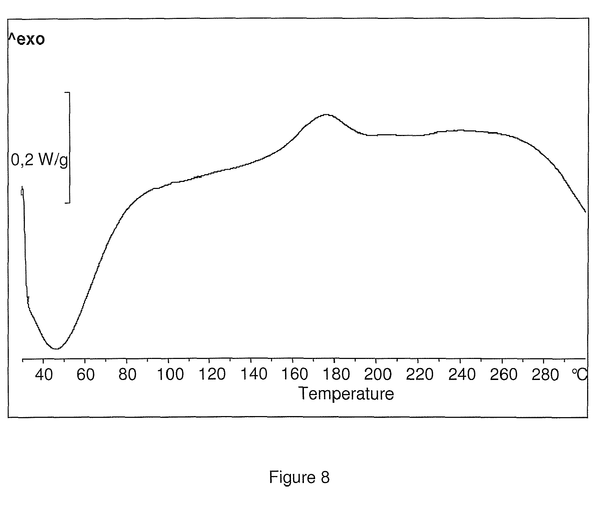

Figure 7 shows a DSC thermogram of hydroxypropyl-β-cyclodextrin alone and Figure 8 shows a DSC thermogram of the inclusion complex between ezetimibe and hydroxypropyl-β-cyclodextrin, prepared according to Example 1.

It can be clearly seen that in the inclusion complex of ezetimibe and hydroxypropyl-β-cyclodextrin, the typical endothermic peak (melting point) is not present. This is evident from thermogram of Figure 8, where a small exothermic peak at around 180ºC is instead shown. This exothermic peak could represent a solid-solid transition in the inclusion complex.

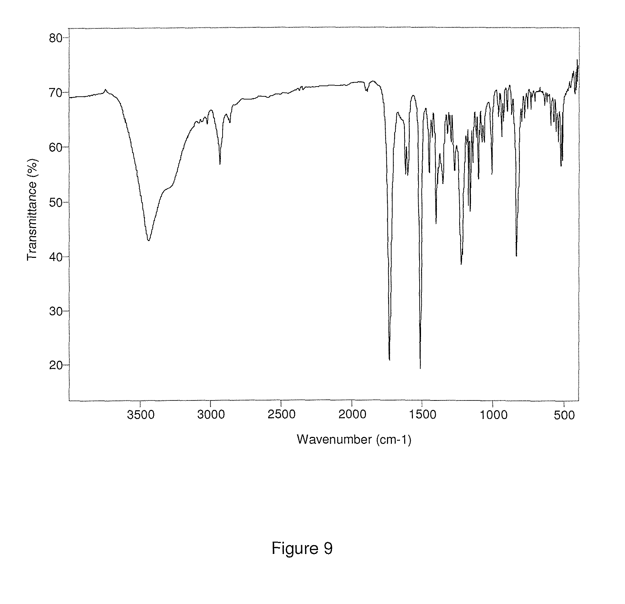

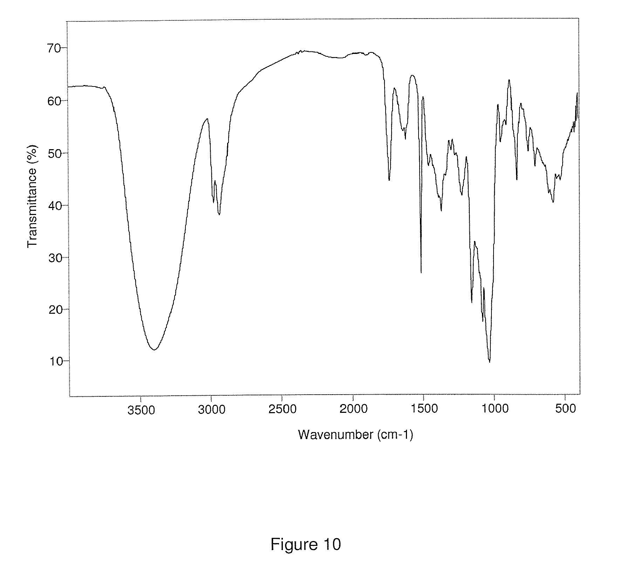

The IR spectrum for crystalline ezetimibe is shown in Figure 9 and the IR spectrum for the inclusion complex of ezetimibe and hydroxypropyl-β-cyclodextrin is shown in Figure 10. The difference between the complexed and un-complexed compounds is clearly seen.

## Claims

1. An inclusion complex of an optionally substituted cyclodextrin and ezetimibe or a pharmaceutically acceptable ester or analogue thereof.

2. An inclusion complex according to Claim 1 wherein the optionally substituted cyclodextrin is a β-cyclodextrin.

3. An inclusion complex according to Claims 1 or Claim 2 wherein the optionally substituted cyclodextrin is hydroxypropyl-β-cyclodextrin.

4. An inclusion complex according to Claim 1 of hydroxypropyl-β-cyclodextrin and ezetimibe.

5. An inclusion complex according to any preceding claim wherein the complex comprises between 1 to 80% ezetimibe by molar percentage, or a pharmaceutically acceptable ester or analogue thereof.

6. An inclusion complex according to any preceding claim wherein the complex comprises between 1 to 50% ezetimibe by molar percentage, or a pharmaceutically acceptable ester or analogue thereof.

7. An inclusion complex according to any preceding claim wherein the complex comprises between 10 to 30% ezetimibe by molar percentage, or a pharmaceutically acceptable ester or analogue thereof.

8. An inclusion complex according to any preceding claim wherein the complex comprises between 15 to 20% ezetimibe by molar percentage, or a pharmaceutically acceptable ester or analogue thereof.

9. An inclusion complex according to any preceding claim wherein the complex comprises 18% ezetimibe by molar percentage, or a pharmaceutically acceptable ester or analogue thereof.

10. An inclusion complex of ezetimibe and hydroxypropyl-β-cyclodextrin **characterized by** at least one of the group consisting of an x-ray diffraction pattern as shown in Figure 1, a solid state ¹³C NMR spectrum as shown in Figure 5, a DSC thermogram as shown in Figure 8, an IR spectrum as shown in Figure 10 or a small exothermic peak at around 180 ºC when measured on a DSC thermogram.

11. A process for the preparation of an inclusion complex of ezetimibe or a pharmaceutically acceptable ester or analogue thereof, and an optionally substituted cyclodextrin comprising:
a) preparation of a solution of ezetimibe or the pharmaceutically acceptable ester or analogue thereof, in a water miscible organic solvent;
b) preparation of a solution of the optionally substituted cyclodextrin in water;
c) combining the prepared solution of ezetimibe or the pharmaceutically acceptable ester or analogue thereof in the water miscible organic solvent, with the solution of the optionally substituted cyclodextrin in water.

12. The process according to Claim 11, wherein the water miscible solvent can be freeze-dried.

13. The process according to Claim 11 or Claim 12 wherein water miscible organic solvent is selected from a group of alcohol and acetone.

14. The process according to claim 13, wherein said alcohol is tert-butanol.

15. The process according to any of claims 11 to 14 wherein the optionally substituted cyclodextrin is β-cyclodextrin.

16. The process according to any of claims 11 to 15 wherein the optionally substituted cyclodextrin is hydroxypropyl-β-cyclodextrin.

17. The process according to any of claims 11 to 16 wherein the ezetimibe or the pharmaceutically acceptable ester or analogue thereof, and the the cyclodextrin are combined in a molar ratio of from 1:1 to 1:2.

18. The process according to any of claims 11 to 17 wherein the ezetimibe or the pharmaceutically acceptable ester or analogue thereof, and the cyclodextrin are combined in a molar ratio of 1:1.3.

19. The process according to any of claims 11 to 18 incorporating at least one of the further steps of:
d) vigorously stirring the formed combined solution; and
e) freezing and freeze drying the solution.

20. A pharmaceutical composition comprising an inclusion complex as claimed in any preceding claim and a pharmaceutically acceptable carrier.

21. A pharmaceutical composition comprising an inclusion complex of hydroxypropyl-β-cyclodextrin and ezetimibe, or a pharmaceutically acceptable ester or analogue thereof, and a pharmaceutically acceptable carrier.

22. Use of an inclusion complex as claimed in any of claims 1 to 10 in the manufacture of a medicament.

23. Use of an inclusion complex as claimed in any of claims 1 to 10 in the manufacture of a medicament for the prophylaxis or treatment of atherosclerotic coronary heart disease.

24. Use of an inclusion complex as claimed in any of claims 1 to 10 in the manufacture of a medicament for the prophylaxis or treatment of coronary heart disease.

25. Use of an inclusion complex as claimed in any of claims 1 to 10 in the manufacture of a medicament for the prophylaxis or treatment of heart disease.

26. Use of an inclusion complex as claimed in any of claims 1 to 10 in the manufacture of a medicament for the inhibition of cholesterol absorption.

27. A method for the prophylaxis or treatment of one at least one of the group consisting of atherosclerotic coronary heart disease, coronary heart disease, or heart disease, in a mammal comprising administering to the affected individual an inclusion complex as claimed in any of claims 1 to 10, or a pharmaceutical composition thereof.

28. A method for inhibiting cholesterol absorption in a patient comprising administering to the affected individual an inclusion complex as claimed in any of claims 1 to 10, or a pharmaceutical composition thereof.

29. An inclusion complex substantially as hereinbefore described and with reference to the accompanying Figures.
